# EUROPEAN PATENT APPLICATION

(11) **EP 4 082 522 A1**
(43) Date of publication of application: **02.11.2022**
(21) Application number: 20908428.4
(22) Date of filing: 16.12.2020
(51) Int. Cl.: A61K 8/898, A61K 8/31, A61K 8/34, A61K 8/37, A61K 8/891, A61K 8/92, A61Q 5/00, A61Q 5/12

(54) **HAIR COSMETIC COMPOSITION**

(30) Priority: 23.12.2019 JP 2019232111
(71) Applicant: Kao Corporation, Chuo-ku Tokyo 103-8210 (JP)
(72) Inventor: SUZUKI, Ryosuke, Tokyo 131-8501 (JP)
(74) Representative: Hoffmann Eitle
(86) International application number: PCT/JP2020/046877
(87) International publication number: WO 2021/131939

(57) **Abstract**

A hair cosmetic composition including the following components (A) and (B), wherein the content of component (A) is 0.05% by mass or more and 10% by mass or less, and the content of component (B) is 5% by mass or more and 99.9% by mass or less: (A) a self-crosslinking compound; and (B) a compound having an IOB of 1.3 or less in the organic conceptual diagram and being liquid at 20°C.

## Description

### Field of the Invention

The present invention relates to a hair cosmetic composition.

### Background of the Invention

In recent years, hair ends, in particular, have been seriously damaged due to chemical treatments such as hair colors and perms and hair set or the like using heat from hair irons, hair dryers, etc., which is now becoming common mainly among young women. It has been reported that damaged hair is associated with loss of 18-MEA (18-methyleicosanic acid), a fatty acid covering the surface of hair, resulting in hydrophilization of the hair surface and an increased surface friction. Symptoms caused by accumulation of such damage, including tangling of hair ends during styling that makes it difficult to create an ideal hair style and poor finger combability are now a serious concern for consumers.

Thus, a technique for recovering hydrophobicity and low friction inherent in healthy hair has been required. Some hair cosmetic compositions which can form a durable polymer coating on the surface of damaged hydrophilic hair to recover lost hydrophobicity have been proposed as an example of such techniques.

For example, Patent Literature 1 discloses a technique in which a copolymer made of (meth)acrylic ester monomers having a silyl group to which a reactive functional group is bonded as a constituent monomer is hydrolyzed and crosslinked on the hair to form a cross-linked coating having excellent resistance to washing.

Furthermore, Patent Literature 2 discloses a technique of forming a hydrophobic coating which is durable against shampooing using a personal care composition comprising a reaction product of a specific oxirane or oxetane compound and a specific amino silane compound (epoxyaminosilane copolymer).

(Patent Literature 1) WO 9854255
(Patent Literature 2) JP-A-2011-503059

### Summary of the Invention

The present invention provides a hair cosmetic composition comprising the following components (A) and (B), wherein the content of component (A) is 0.05% by mass or more and 10% by mass or less, and the content of component (B) is 5% by mass or more and 99.9% by mass or less:
(A) a self-crosslinking compound; and
(B) a compound having an IOB of 1.3 or less in the organic
conceptual diagram and being liquid at 20°C.

The present invention also provides a method of using a hair cosmetic composition, comprising applying the composition to hair and then drying the hair without rinsing off.

### Detailed Description of the Invention

In the technique described in Patent Literature 1, the composition needs to be separated from water before use in order to prevent the copolymer from being cross-linked during storage. Thus, a hydrolyzation step for cross-linking must be performed immediately before use, and this cannot be said to be convenient for consumers.

Furthermore, although the technique described in Patent Literature 2 is more convenient, durability against washing is achieved only through a process in which hair is subjected to an immersion treatment at a high bath ratio and dried for enough time, and thus no effect is obtained in a leave-on treatment with a low bath ratio. Accordingly, this does not meet recent consumers' demand to have effects in a simple, short-time treatment using a small amount of compositions.

Accordingly, the present invention relates to a hair cosmetic composition which gives sufficient hydrophobicity, has friction-lowering effects when hair is treated therewith for a short time at a low bath ratio, provides easy untangling of tress in drying step, good finger combability during drying and little stickiness of hair to fingers after drying, makes hair manageable after drying, and can maintain such effects for a long time after the treatment even when repeating shampooing.

The present inventors conducted intensive studies and found that the above problem can be solved by using a self-crosslinking compound and a compound having an IOB of 1.3 or less in the organic conceptual diagram and being liquid at 20°C, in combination, and completed the present invention.

The hair cosmetic composition of the present invention gives sufficient hydrophobicity, has friction-lowering effects when hair is treated therewith for a short time at a low bath ratio, provides easy untangling of tress in drying step, good finger combability during drying and little stickiness of hair to fingers after drying, makes hair manageable after drying, and can maintain such effects for a long time after the treatment even when repeating shampooing.

### [component (A): self-crosslinking compound]

The self-crosslinking compound of the component (A) contains the following components (A1) and (A2).
(A1) an alkoxysilyl group-containing silicone
(A2) an alkoxysilyl group-containing alkylamine

Both the components (A1) and (A2) have an alkoxysilyl group - SiR¹ₙ(OR²)₃₋ₙ wherein R¹ and R² independently represent a monovalent hydrocarbon group and n represents an integer of 0 to 2.

The alkoxysilyl group-containing silicone of the component (A1) is a silicone in which the alkoxysilyl group described above is bonded to an organopolysiloxane residue.

The organopolysiloxane residue has an amino group, and more preferably an amino group and a polyether group. Specific examples of components (A1) include the following epoxyaminosilane copolymer and (amodimethicone/ morpholinomethyl silsesquioxane) copolymer. The epoxyaminosilane copolymer is preferred.

The epoxyaminosilane copolymer is a reaction product of the following compounds (a) to (d).
(a) a polysiloxane having at least two oxiranyl groups or oxetanyl groups;
(b) a polyether having at least two oxiranyl groups or oxetanyl groups;
(c) aminopropyltrialkoxysilane; and
(d) a compound selected from the group consisting of the following primary and secondary amines:
   - primary amines: methylamine, ethylamine, propyleneamine, ethanolamine, isopropylamine, butylamine, isobutylamine, hexylamine, dodecylamine, oleylamine, aniline, aminopropyltrimethylsilane, aminopropyltriethylsilane, aminomorpholine, aminopropyldiethylamine, benzylamine, naphthylamine, 3-amino-9-ethylcarbazole, 1-aminoheptafluorohexane, and 2,2,3,3,4,4,5,5,6,6,7,7,8,8,8-pentadecafluoro-1-octanamine; and
   - secondary amines: methylethylamine, methyloctadecylamine, diethanolamine, dibenzylamine, dihexylaminedicyclohexylamine, piperidine, pyrrolidinephthalimide, and a polymer amine.

### <Compounds (a) and (b)>

The compound (a) is a polysiloxane having at least two oxiranyl groups or oxetanyl groups. Examples thereof include those of the following formula (1): 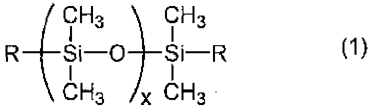 wherein R represents a hydrocarbon group having 1 to 6 carbon atoms and having an oxiranyl group or an oxetanyl group at the terminal thereof and optionally having a heteroatom, and x represents a number of from 1 to 1,000.

The compound (b) is a polyether having at least two oxiranyl groups or oxetanyl groups. Examples thereof include those of the following formula (2): 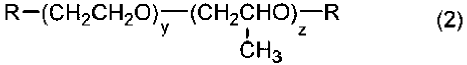 wherein R is as defined above, y represents a number of from 1 to 100, z represents a number of from 0 to 100, and y + z is 1 to 200.

In the formulas (1) and (2), an oxygen atom is preferred as the heteroatom which R optionally has. Examples of R include an oxiranylmethyl group (glycidyl group), an oxiranylmethoxy group (glycidyloxy group), an oxiranylmethoxypropyl group (glycidyloxypropyl group), an oxetanylmethyl group, an oxetanylmethoxy group, an oxetanylmethoxypropyl group and a 3-ethyloxetanylmethyl group. In particular, a hydrocarbon group having 1 to 4 carbon atoms and an oxiranyl group and optionally having an oxygen heteroatom is preferred, and at least one selected from an oxiranylmethyl group (glycidyl group), an oxiranylmethoxy group (glycidyloxy group) and an oxiranylmethoxypropyl group (glycidyloxypropyl group) is more preferred.

### <Compound (c)>

The compound (c) is aminopropyltrialkoxysilane. Examples of alkoxy groups in the compound (c) include those having 1 to 6 carbon atoms. Those having 2 to 4 carbon atoms are preferred and those having 3 carbon atoms are more preferred, and an isopropoxy group is particularly preferred. Examples of compounds (c) include aminopropyltrimethoxysilane, aminopropyltriethoxysilane, aminopropyltripropoxysilane, aminopropyltriisopropoxysilane, aminopropyltributoxysilane and aminopropyltri-tert-butoxysilane. Among them, aminopropyltriisopropoxysilane is preferred. For compound (c), one of these may be used alone, or two or more of them may be used in combination.

### <Compound (d)>

The compound (d) is a compound selected from the group consisting of the following primary and secondary amines:
- primary amines: methylamine, ethylamine, propyleneamine, ethanolamine, isopropylamine, butylamine, isobutylamine, hexylamine, dodecylamine, oleylamine, aniline, aminopropyltrimethylsilane, aminopropyltriethylsilane, aminomorpholine, aminoethyldimethylamine, aminoethyldiethylamine, aminoethyldibutylamine, aminopropyldimethylamine, aminopropyldiethylamine, aminopropyldibutylamine, benzylamine, naphthylamine, 3-amino-9-ethylcarbazole, 1-aminoheptafluorohexane, and 2,2,3,3,4,4,5,5,6,6,7,7,8,8,8-pentadecafluoro-1-octanamine; and
- secondary amines: methylethylamine, methyloctadecylamine, diethanolamine, dibenzylamine, dihexylamine, dicyclohexylamine, piperidine, pyrrolidinephthalimide, and a polymer amine.

Among them, primary amines are preferred, and at least one selected from aminopropyldiethylamine, aminopropyldimethylamine and aminopropyldibutylamine is more preferred. For compound (d), one of these may be used alone, or two or more of them may be used in combination.

The reaction of the compounds (a) to (d) is performed under reflux in a solvent such as isopropanol for a predetermined time. The molar ratio of the oxiranyl groups or the oxetanyl groups in the compound (a) and (b) to the amino group in the compound (c) is preferably 1 or more, more preferably 1.1 or more, and further preferably 1.2 or more, and preferably 4 or less, more preferably 3.9 or less, and further preferably 3.8 or less.

Examples of epoxyaminosilane copolymers of the component (A1) include those with the INCI name of Polysilicone-29, which is Silsoft CLX-E (manufactured by Momentive Performance Materials; containing 15% by mass of an active ingredient, dipropylene glycol and water). Examples of amodimethicone/ morpholinomethyl silsesquioxane copolymers of the component (A1) include Belsil ADM 6300, the same 8301 (manufactured by Wacker Asahikasei Silicone, Co., Ltd.)

The alkoxysilyl group-containing alkylamine of the component (A2) is a compound in which the alkoxysilyl group described above is bonded to a group of the following formula (3). 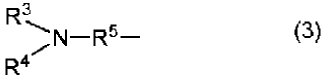 wherein R³ and R⁴ represent a hydrogen atom or a hydrocarbon group optionally substituted with an amino group, or the two may be bonded to form an alkylidene group, and R⁵ represents a divalent hydrocarbon group having 1 to 6 carbon atoms.

R⁵ in the formula (3) is preferably a group having 2 to 4 carbon atoms. A trimethylene group is particularly preferred. Examples of components (A2) include N-2-(aminoethyl)-3-aminopropylmethyldimethoxysilane (KBM-602; manufactured by Shin-Etsu Chemical Co., Ltd.), N-2-(aminoethyl)-3-aminopropyltrimethoxysilane (KBM-603; manufactured by Shin-Etsu Chemical Co., Ltd.), 3-aminopropyltrimethoxysilane (KBM-903; manufactured by Shin-Etsu Chemical Co., Ltd.), 3-aminopropyltriethoxysilane (KBE-903; manufactured by Shin-Etsu Chemical Co., Ltd.), 3-triethoxysilyl-N-(1,3-dimethylbutylidene)propylamine (KBE-9103P; manufactured by Shin-Etsu Chemical Co., Ltd.), N-phenyl-3-aminopropyltrimethoxysilane (KBM-573; manufactured by Shin-Etsu Chemical Co., Ltd.), and hydrochloride of N-(vinylbenzyl)-2-aminoethyl-3-aminopropyltrimethoxysilane (KBM-575; manufactured by Shin-Etsu Chemical Co., Ltd.). Of them, 3-aminopropyl-triethoxysilane, 3-aminopropylmethyldiethoxysilane, N-(2-aminoethyl)-3-aminopropyltriethoxysilane, and 3-(2-aminoethylamino)propylmethyldiethoxysilane are particularly preferred.

The content of component (A) in the hair cosmetic composition of the present invention is 0.05% by mass or more, preferably 0.1% by mass or more, more preferably 0.2% by mass or more, and further preferably 0.25% by mass or more to give sufficient hydrophobicity to hair, have friction-lowering effects, provide easy untangling of tress in drying step, good finger combability during drying, little stickiness of hair to fingers after drying, make hair manageable after drying, and maintain such effects after repeating shampooing. The content of component (A) is 10% by mass or less, preferably 7.5% by mass or less, more preferably 5% by mass or less, further preferably 2.5% by mass or less, and further more preferably 1.5% by mass or less to avoid giving sticky feeling, maintain friction-lowering effects after repeating shampooing and provide easy untangling of tress in drying step and good finger combability during drying.

### [Component (B): Compound having IOB of 1.3 or less in organic conceptual diagram and being liquid at 20°C]

The component (B) is a compound having an IOB of 1.3 or less in the organic conceptual diagram and being liquid at 20°C. In the present invention, being liquid at 20°C means that the component has a viscosity at 20°C of 10,000 mPa · s or less. The viscosity is measured by using a B-type viscometer (Vismetron viscometer: Model No. VS-A1 manufactured by Shibaura Systems Co., Ltd.) under conditions of 1 atm and 35°C with spindle No. 3 at 12 rpm (rotation/ minute) for 30 seconds.

The organic conceptual diagram is proposed by Atsushi Fujita and details of the diagram are described in, for example, "Pharmaceutical Bulletin," vol. 2, 2, pp. 163-173 (1954), "Journal of Japanese Chemistry," vol. 11, 10, pp. 719-725 (1957), "Fragrance Journal," vol. 50, pp. 79-82 (1981) and "Organic Conceptual Diagram, Basis and Application, " (Yoshio Koda, Sankyo Publishing, 1984). More specifically, all organic compounds are regarded as a derivative of methane (CH₄) and a fixed numerical value is assigned to the number of carbon atoms, the substituents, the modified moieties, the rings and the like of the organic compound, and the values are added to determine an organic value and an inorganic value, and they are plotted with the organic values on the X axis and the inorganic values on the Y axis.

IOB in the organic conceptual diagram means the ratio of the inorganic value (IV) to the organic value (OV) in the organic conceptual diagram, i.e., "inorganic value (IV)/ organic value (OV)."

Examples of liquid compounds corresponding to the component (B) include isododecane (0), hydrogenated polyisobutene (0), mineral oil (0), jojoba oil (0.07), octyldodecyl isostearate (0.08), octyldodecyl neopentanoate (0.13), toriisostearin (0.16), isodecyl neopentanoate (0.22), dimethylpolysiloxane(0.3 to 0.5), phenyltrimethicone (0.28), methyltrimethicone (0.175), isopropyl palmitate (0.162), hydrogenated castor oil (0.42), dimethyl ether(0.50), glyceryl isostearate (0.63), benzyl alcohol (0.82), phenoxyethanol (0.844), PEG 20 hydrogenated castor oil (0.94), PEG 25 hydrogenated castor oil (1.02), PEG 30 hydrogenated castor oil (1.10) and PEG 45 hydrogenated castor oil (1.26) (the numerical value in the parenthesis indicates IOB). Of them, at least one selected from the group consisting of isododecane, hydrogenated polyisobutene, dimethylpolysiloxane, phenyl trimethicone, methyl trimethicone, isopropyl palmitate, benzyl alcohol and phenoxy ethanol is preferred.

When the hair cosmetic composition of the present invention does not include the component (C), the content of the component (B) in the hair cosmetic composition of the present invention is 5% by mass or more, preferably 15% by mass or more, and more preferably 20% by mass or more to give sufficient hydrophobicity to hair, have friction-lowering effects, provide easy untangling of tress in drying step, good finger combability during drying, little stickiness of hair to fingers after drying, make hair manageable after drying, and maintain such effects after repeating shampooing. The content is 99.9% by mass or less, preferably 99.0% by mass or less, and more preferably 95.0% by mass or less for the same reason. When the hair cosmetic composition of the present invention contains the component (C), the content of the component (B) in the hair cosmetic composition of the present invention is 5% by mass or more, preferably 7.5% by mass or more, more preferably 15% by mass or more, and further preferably 25% by mass to give sufficient hydrophobicity to hair, have friction-lowering effects, provide easy untangling of tress in drying step, good finger combability during drying, little stickiness of hair to fingers after drying, make hair manageable after drying, and maintain such effects after repeating shampooing. The content is preferably 75% by mass or less, more preferably 45% by mass or less, and further preferably 35% by mass or less from the same point of view.

### [Component (C): Aliphatic alcohol having 1 to 4 carbon atoms]

It is preferable that the hair cosmetic composition of the present invention further contains an aliphatic alcohol having 1 to 4 carbon atoms as a component (C). Examples of components (C) include methanol, ethanol, 1-propanol, 2-propanol and butanol. Of them, ethanol is preferred.

The content of the component (C) in the hair cosmetic composition of the present invention is preferably 10% by mass or more, more preferably 15% by mass or more, and further preferably 20% by mass or more to make application of the composition to hair easy, give sufficient hydrophobicity to hair, have friction-lowering effects, provide easy untangling of tress in drying step, good finger combability during drying, little stickiness of hair to fingers after drying, make hair manageable after drying, and maintain such effects after repeating shampooing. The content of the component (C) is preferably 90% by mass or less, more preferably 85% by mass or less, further preferably 80% by mass or less, and further more preferably 70% by mass or less to improve storage stability, give sufficient hydrophobicity to hair, have friction-lowering effects, provide easy untangling of tress in drying step, good finger combability during drying, little stickiness of hair to fingers after drying, make hair manageable after drying, and maintain such effects after repeating shampooing.

### [Water]

The hair cosmetic composition of the present invention may also contain water as a solvent. The content of water in the hair cosmetic composition of the present invention is 13% by mass or less, preferably 10.5% by mass or less, more preferably 8% by mass or less, and further preferably 5.5% by mass or less to make the present invention more effective.

### [Thickener]

The hair cosmetic composition of the present invention may also contain a thickener. Either of an aqueous thickener or an oil thickener may be used. Examples of aqueous thickeners include an anionic thickener, a cationic thickener and a nonionic thickener.

Specific examples of anionic thickeners include a polyacrylic acid (Carbopol 941, ditto 981 manufactured by Noveon), an acrylic acid alkyl methacrylate copolymer (Carbopol ETD 2020 manufactured by Noveon), a hydrolysate of a lower alkyl vinyl ether/maleic anhydride copolymer partially cross-linked with a terminal-unsaturated diene compound, or a monoalkyl ester thereof (Stabilieze 06, ditto QM manufactured by ASHLAND), carrageenan (e.g., SOAGEENA LX22, ditto ML210 manufactured by Mitsubishi Rayon, Co., Ltd.), xanthan gum (Eco gum T manufactured by Sumitomo Dainippon Pharma Co., Ltd.), welan gum (e.g., K1C376, K1A96 manufactured by Sansho Co., Ltd.), hydroxypropyl xanthan gum (e.g., Rhaball gum EX manufactured by Sumitomo Dainippon Pharma Co., Ltd.), sodium stearoxy PG-hydroxyethylcellulose sulfonate and a hydroxyethyl acrylate/Na acryloyldimethyltaurine) copolymer (e.g., SIMULGEL NS, SEPINOV EMT10 manufactured by SEPPIC).

Examples of cationic thickeners include natural or semi-synthetic cationic polysaccharides and synthetic polymers having an amino group or an ammonium group in the side chain of polymer chains, or having a diallyl quaternary ammonium salt as a structural unit.

Specific examples of cationic polysaccharides include cationized cellulose derivatives (e.g., LEOGARD G, ditto GP manufactured by Lion Corporation, UCARE polymer JR-125, ditto JR-400, JR-30M, LR-400, LR-30M manufactured by The Dow Chemical Company, CELQUAT H-100, ditto L-200 manufactured by AkzoNovel), cationized guar gum derivatives (e.g., JAGUAR C-13S, ditto C-17 manufactured by Solvay, Rhaball gum CG-M, ditto CG-M7, CG-M8M manufactured by DSP GOKYO FOOD & CHEMICAL Co., Ltd.), hydroxypropyl chitosan (e.g. Chitofilmer HV-10 manufactured by ICHIMARU PHARCOS) and chitosan · dl-pyrrolidone carboxylate (e.g., KYTAMER manufactured by Union Carbide Corporation).

Examples of synthetic cationic polymers having an amino group or an ammonium group in the side chain of polymer chains include a synthetic cationic polymer containing trialkylaminoalkyl (meth)acrylate, trialkylaminoalkyl (meth)acrylamide, (meth)acrylamide, or vinylamine as a structural unit. Specific examples thereof include poly(ethyltrimonium chloride methacrylate) (INCI name: Polyquaternium-37, e.g., Cosmedia Ultragel 300 manufactured by BASF), an (acrylic acid/methyl acrylate/3-methacryloylaminopropyl trimethylammonium chloride) copolymer (INCI Name: Polyquaternium-47, e.g., Merquat 2201 manufactured by Lubrizol), an (acrylic acid/acrylamide/methylmethacrylamidopropyltrimethylammonium chloride) copolymer (INCI Name: Polyquaternium-53, e.g., Merquat 2003 manufactured by Lubrizol), a (dimethylacrylamide/ethyl methacrylate trimonium chloride) copolymer (e.g., Tinobis CD manufactured by BASF), and a (vinyl amine/vinyl alcohol) copolymer (e.g., SEVOL ULTALUX AD manufactured by Sekisui Specialty Chemicals, Diafix C-601 manufactured by Mitsubishi Chemical Corporation).

Specific examples of synthetic cationic polymers having a diallyl quaternary ammonium salt as a structural unit include a polymer of diallyl dimethyl ammonium chloride (INCI Name: Polyquaternium-6, e.g., Merquat 100 manufactured by Lubrizol), a (dimethyldiallylammonium chloride/acrylamide) copolymer (INCI: Polyquaternium-7, e.g., Merquat 550, ditto 740 manufactured by Lubrizol), an (acrylic acid/diallyl dimethyl ammonium chloride) copolymer (INCI Name: Polyquaternium-22, e.g., Merquat 280, ditto 295 manufactured by Lubrizol) and an (acrylamide/acrylic acid/diallyl dimethyl ammonium chloride) copolymer (INCI Name: Polyquaternium-39, e.g., Merquat Plus 3330, ditto 3331 manufactured by Lubrizol).

Examples of nonionic thickening polymers include natural or semi-synthetic nonionic polysaccharides and synthetic nonionic polymers having vinyl alcohol or oxyalkylene as a structural unit.

Specific examples of natural or semi-synthetic nonionic polysaccharides include water-soluble natural polysaccharides such as starch, guar gum, locust bean gum and glucomannan, and water-soluble hydroxyalkylated polysaccharides prepared by reacting an alkylene oxide with cellulose, starch, guar gum, locust bean gum or the like. Specific examples thereof include guar gum (e.g., Fiberon S manufactured by DSP GOKYO FOOD & CHEMICAL Co., Ltd.) and pullulan (pullulan PI-20 manufactured by Hayashibara Co., Ltd.). Examples include hydroxyethylcellulose (e.g. SE-850 manufactured by DAICEL FINECHEM Ltd., CELLOSIZE HEC QP-52000-H manufactured by The Dow Chemical Company), methyl hydroxyethylcellulose (STRUCTURE CELL 12000M manufactured by AkzoNovel), hydroxypropylcellulose (e.g. HPC-H, ditto HPC-M, HPC-L manufactured by NIPPON SODA CO., LTD.) and hydroxypropyl methylcellulose (e.g. METOLOSE 60SH-10000 manufactured by Shin-Etsu Chemical Co., Ltd.).

Specific examples of synthetic nonionic thickening polymers having vinyl alcohol or oxyalkylene as a structural unit include polyvinyl alcohol (e.g., GOHSENOL EG-40, ditto GH-05, KH-20, NH-26 manufactured by The Nippon Synthetic Chemical Co., Ltd.), a highly polymerized polyethylene glycol (e.g., POLYOX WSR N-60K, ditto WSR301, WSR303 manufactured by The Dow Chemical Company) and a (PEG-240/decyltetradeceth-20/HDI) copolymer (e.g., ADEKA NOL GT-700 manufactured by ADEKA Corporation).

Examples of oil thickeners include organic oil thickeners such as a sucrose ester of fatty acid, solid oil, metal soap and 12-hydroxystearic acid, and inorganic polymers such as bentonite and hectorite.

Examples of sucrose esters of fatty acid include esters of fatty acid having 10 to 22 carbon atoms with dextrin, sucrose or inulin. Specific examples thereof include dextrin palmitate, dextrin myristate, dextrin (palmitate/ ethylhexanoate), dextrin stearate, dextrin behenate, dextrin laurate, dextrin cocoate, sucrose palmitate, sucrose stearate and stearoyl inulin. Examples of commercially available products include Rheopearl KL2, Rheopearl TL2, Rheopearl TT2, Rheopearl MKL2 and Rheopearl ISK2 (all manufactured by Chiba Flour Milling Co., Ltd.).

Examples of solid oil include paraffin, ceresin, synthetic hydrocarbon wax, Fisher-Tropsch wax, microcrystalline wax, polyethylene wax, ethylene propylene copolymer, candelilla wax, beeswax and carnauba wax.

One of these thickeners may be used alone, or two or more of them may be used in combination. The content of the thickener in the hair cosmetic composition of the present invention is preferably 0.01% by mass or more, more preferably 0.05% by mass or more, and further preferably 0.10% by mass or more, and preferably 5.0% by mass or less, more preferably 3.0% by mass or less, and further preferably 1.0% by mass or less from the viewpoint of appropriate and easy application to hair.

### [Optional components]

The hair cosmetic composition of the present invention may also contain, in addition to the above components, a component usually blended in a hair cosmetic composition. Examples thereof include an anti-dandruff agent; a vitamin preparation; a microbicide; an anti-inflammatory agent; an antiseptic; a chelating agent; a moisturizer; a colorant such as a dye or a pigment; extracts; a pearling agent; a perfume; an ultraviolet absorber; an antioxidant; a photocatalyst; shea butter; rose water; sunflower oil; orange oil; eucalyptus oil; and a surfactant. Examples of photocatalysts include metal oxides such as titanium oxide and tungsten oxide, aromatic hydroxy compounds such as 8-hydroxyquinoline, 7-cyano-2-naphthol and 8-quinolinol-1-oxide, sulfonated pyrene compounds, onium salts, diazomethane derivatives, bissulfone derivatives, disulfono derivatives, nitrobenzyl sulfonate derivatives, sulfonic acid ester derivatives, and a sulfonic acid ester of N-hydroxyimide. Any one of a cationic surfactant, an anionic surfactant, an amphoteric surfactant and a nonionic surfactant may be used as the surfactant. Examples of cationic surfactants include an alkylamine salt and an alkyl quaternary ammonium salt. Examples of anionic surfactants include an alkyl sulfonate, an alkyl carboxylate, an alkyl ether sulfonate and an alkyl ether carboxylate. Examples of amphoteric surfactants include imidazoline, carbobetaine, amidobetaine, sulfobetaine, hydroxysulfobetaine and amidosulfobetaine. Examples of nonionic surfactants include esters such as a glycerol fatty acid ester, a sorbitan fatty acid ester and a sucrose fatty acid ester, and ethers such as a polyoxyethylene alkyl ether, a polyoxyethylene alkylphenyl ether, a polyoxyethylene · polyoxypropylene alkyl ether and polyoxyethylene · polyoxypropylene alkyl phenyl ether.

More specifically, polyols such as propylene glycol, dipropylene glycol and glycerol may be used for the purpose of, for example, moisturization. The content of these components in the hair cosmetic composition of the present invention is preferably 20% by mass or less, more preferably 10% by mass or less, and further preferably 5% by weight or less. The amount of the photocatalyst in the hair cosmetic composition of the present invention is preferably 2% by mass or less, more preferably 1% by mass or less, further preferably 0.1% by weight or less, and still more preferably substantially 0% by mass from the viewpoint of maintaining storage stability of the cosmetic composition. The amount of the surfactant in the hair cosmetic composition of the present invention is preferably 2% by mass or less, more preferably 1% by mass or less, further preferably 0.1% by weight or less, and still more preferably substantially 0% by mass from the viewpoint of maintaining persistent effects.

### [Method of use]

The hair cosmetic composition of the present invention may be used by a method in which the composition is rinsed off after being applied to hair, or a method in which the composition is applied to hair and dried without being rinsed off. It is preferable that the composition be used by the method in which the composition is applied to hair and then dried without being rinsing off in order to increase the effect of the present invention.

The amount of the hair cosmetic composition of the present invention to be applied to hair is determined relative to the mass of the hair so that the bath ratio (the mass of the hair cosmetic composition/the mass of the hair) is preferably 0.001 or more, more preferably 0.005 or more, and further preferably 0.01 or more, and preferably 100 or less, more preferably 10 or less, and further preferably 1 or less.

For the embodiment described above, preferred aspects of the present invention will be further disclosed.
<1> A hair cosmetic composition comprising the following components (A) and (B), wherein the content of component (A) is 0.1% by mass or more and 5.0% by mass or less, and the content of component (B) is 10.0% by mass or more and 99.9% by mass or less:
   (A) a self-crosslinking compound; and
   (B) a compound having an IOB of 1.3 or less in the organic
   conceptual diagram and being liquid at 20°C.
<2> The hair cosmetic composition according to claim 1, wherein the component (A) is preferably one or more selected from the group consisting of the following components (A1) and (A2):
   (A1) an alkoxysilyl group-containing silicone; and
   (A2) an alkoxysilyl group-containing alkylamine.
<3> The hair cosmetic composition according to <2>, wherein the component (A1) is preferably silicone in which an alkoxysilyl group - SiR¹ₙ(OR²)₃₋ₙ (wherein R¹ and R² independently represent a monovalent hydrocarbon group and n represents an integer of 0 to 2) is bonded to an organopolysiloxane residue.
<4> The hair cosmetic composition according to <3>, wherein the component (A1) is preferably an epoxyaminosilane copolymer, which is a reaction product of the following compounds (a) to (d):
   (a) a polysiloxane having at least two oxiranyl groups or oxetanyl groups;
   (b) a polyether having at least two oxiranyl groups or oxetanyl groups;
   (c) aminopropyltrialkoxysilane; and
   (d) a compound selected from the group consisting of the following primary and secondary amines:
      - primary amines: methylamine, ethylamine, propyleneamine, ethanolamine, isopropylamine, butylamine, isobutylamine, hexylamine, dodecylamine, oleylamine, aniline, aminopropyltrimethylsilane, aminopropyltriethylsilane, aminomorpholine, aminopropyldiethylamine, benzylamine, naphthylamine, 3-amino-9-ethylcarbazole, 1-aminoheptafluorohexane, and 2,2,3,3,4,4,5,5,6,6,7,7,8,8,8-pentadecafluoro-1-octanamine; and
      - secondary amines: methylethylamine, methyloctadecylamine, diethanolamine, dibenzylamine, dihexylamine, dicyclohexylamine, piperidine, pyrrolidinephthalimide, and a polymer amine.
<5> The hair cosmetic composition according to <4>, wherein the compound (a) is preferably a compound of the following formula (1): 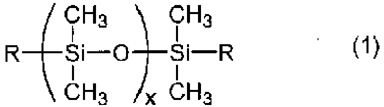 wherein R represents a hydrocarbon group having 1 to 6 carbon atoms and having an oxiranyl group or an oxetanyl group at the terminal thereof and optionally having a heteroatom, and x represents a number of from 1 to 1,000.
<6> The hair cosmetic composition according to <4> or <5>, wherein the compound (b) is preferably a compound of the following formula (2): 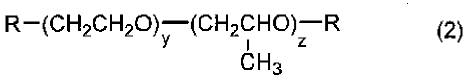 wherein R is as defined above, y represents a number of from 1 to 100, z represents a number of from 0 to 100, and y + z is 1 to 200.
<7> The hair cosmetic composition according to any one of <4> to <6>, wherein the compound (c) is preferably at least one selected from the group consisting of aminopropyltrimethoxysilane, aminopropyltriethoxysilane, aminopropyltripropoxysilane, aminopropyltriisopropoxysilane, aminopropyltributoxysilane and aminopropyltri-tert-butoxysilane.
<8> The hair cosmetic composition according to any one of <4> to <7>, wherein compound (d) is preferably a primary amine, and more preferably at least one selected from aminopropyldiethylamine, aminopropyldimethylamine and aminopropyldibutylamine.
<9> The hair cosmetic composition according to any one of <4> to <8>, wherein component (A1) is preferably polysilicone-29.
<10> The hair cosmetic composition according to <3>, wherein the component (A1) is preferably an (amodimethicone/ morpholinomethyl silsesquioxane) copolymer.
<11> The hair cosmetic composition according to <2>, wherein the component (A2) is preferably a compound in which an alkoxysilyl group - SiR¹ₙ(OR²)₃₋ₙ (wherein R¹ and R² independently represent a monovalent hydrocarbon group and n represents an integer of 0 to 2) is bonded to a group of the following formula (3): 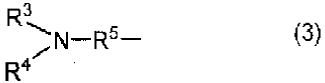 wherein R³ and R⁴ represent a hydrogen atom or a hydrocarbon group optionally substituted with an amino group, or the two may be bonded to form an alkylidene group, and R⁵ represents a divalent hydrocarbon group having 1 to 6 carbon atoms.
<12> The hair cosmetic composition according to <11>, wherein the component (A2) is preferably one or more selected from the group consisting of N-2-(aminoethyl)-3-aminopropylmethyldimethoxysilane, N-2-(aminoethyl)-3-aminopropyltrimethoxysilane, 3-aminopropyltrimethoxysilane, 3-aminopropyltriethoxysilane, 3-triethoxysilyl-N-(1,3-dimethyl-butylidene)propylamine, N-phenyl-3-aminopropyltrimethoxysilane, and hydrochloride of N-(vinylbenzyl)-2-aminoethyl-3-aminopropyltrimethoxysilane, and more preferably at least one selected from the group consisting of 3-aminopropyltriethoxysilane, 3-aminopropylmethyldiethoxysilane, N-2-(aminoethyl)-3-aminopropyltriethoxysilane and 3-(2-aminoethylamino)propylmethyldiethoxysilane.
<13> The hair cosmetic composition according to any one of <1> to <12>, wherein the content of component (A) is 0.1% by mass or more, more preferably 0.2% by mass or more, further preferably 0.25% by mass or more, and preferably 7.5% by mass or less, more preferably 5% by mass or less, and further preferably 2.5% by mass or less, and still more preferably 1.5% by mass or less.
<14> The hair cosmetic composition according to any one of <1> to <13>, wherein the component (B) is preferably at least one selected from the group consisting of isododecane, hydrogenated polyisobutene, mineral oil, jojoba oil, octyldodecyl isostearate, octyldodecyl neopentanoate, toriisostearin, isodecyl neopentanoate, dimethylpolysiloxane, phenyltrimethicone, methyltrimethicone, isopropyl palmitate, hydrogenated castor oil, dimethyl ether, glyceryl isostearate, benzyl alcohol, phenoxyethanol, PEG 20 hydrogenated castor oil, PEG 25 hydrogenated castor oil, PEG 30 hydrogenated castor oil and PEG 45 hydrogenated castor oil, and more preferably at least one selected from the group consisting of isododecane, hydrogenated polyisobutene, dimethylpolysiloxane, phenyltrimethicone, methyltrimethicone, isopropyl palmitate, benzyl alcohol and phenoxyethanol.
<15> The hair cosmetic composition according to any one of <1> to <14>, wherein the composition does not comprise the component (C) and the content of the component (B) is 5% by mass or more, preferably 15% by mass or more, and more preferably 20% by mass, and 99.9% by mass or less, preferably 99.0% by mass or less, and more preferably 95.0% by mass or less.
<16> The hair cosmetic composition according to any one of <1> to <14>, wherein the composition comprises the component (C) and the content of the component (B) is 5% by mass or more, preferably 7.5% by mass or more, more preferably 15% by mass, and further preferably 25% by mass or more, and preferably 75% by mass or less, more preferably 45% by mass or less, and further preferably 35% by mass or less.
<17> The hair cosmetic composition according to any one of <1> to <14>, <16>, preferably further comprising the following component (C):
   (C) an aliphatic alcohol having 1 to 4 carbon atoms.
<18> The hair cosmetic composition according to <17>, wherein the component (C) is preferably selected from the group consisting of methanol, ethanol, 1-propanol, 2-propanol and butanol, and the component (C) is more preferably ethanol.
<19> The hair cosmetic composition according to <17> or <18>, wherein the content of the component (C) is preferably 10% by mass or more, more preferably 15% by mass or more, and further preferably 20% by mass or more, and preferably 90% by mass or less, more preferably 85% by mass or less, and further preferably 80% by mass or less.
<20> The hair cosmetic composition according to any one of <1> to <19>, wherein the content of water is preferably 13% by mass or less, more preferably 10.5% by mass or less, further preferably 8% by mass or less, and further more preferably 5.5% by mass or less.
<21> The hair cosmetic composition according to any one of <1> to <20>, preferably further comprising a thickener.
<22> The hair cosmetic composition according to <21>, wherein the content of the thickener is preferably 0.01% by mass or more, more preferably 0.05% by mass or more, and further preferably 0.10% by mass or more, and preferably 5.0% by mass or less, more preferably 3.0% by mass or less, and further preferably 1.0% by mass or less.
<23> A method of using the hair cosmetic composition according to any one of <1> to <22>, comprising applying the hair cosmetic composition to hair and then drying the hair without rinsing off.
<24> The method of using the hair cosmetic composition according to <23>, wherein the amount of the hair cosmetic composition to be applied to hair is determined relative to the mass of the hair so that the bath ratio is preferably 0.001 or more, more preferably 0.005 or more, and further preferably 0.01 or more, and preferably 100 or less, more preferably 10 or less, and further preferably 1 or less.
<25> A hair cosmetic composition comprising the following components (A) and (B), wherein the content of component (A) is 0.25% by mass or more and 1.5% by mass or less, and the content of component (B) is 20% by mass or more and 99.9% by mass or less:
   (A) a self-crosslinking compound; and
   (B) a compound having an IOB of 1.3 or less in the organic
   conceptual diagram and being liquid at 20°C.
<26> A hair cosmetic composition comprising the following components (A), (B) and (C), wherein the content of component (A) is 0.25% by mass or more and 1.5% by mass or less, the content of component (B) is 25% by mass or more and 35% by mass or less, and the content of component (C) is 20% by mass or more and 70% by mass or less:
   (A) a self-crosslinking compound;
   (B) a compound having an IOB of 1.3 or less in the organic conceptual diagram and being liquid at 20°C; and
   (C) an aliphatic alcohol having 1 to 4 carbon atoms.

### Examples

Examples 1 to 21, Comparative Examples 1 to 5

Oily hair cosmetic compositions having the composition shown in Tables 1 and 2 were prepared, and the advancing contact angle of the hair treated with the compositions with water and combing force of the hair were measured, and various sensory evaluations were performed.

### (Method of treatment)

Damaged hair prepared by bleaching a 5.0-g hair tress of the hair from a healthy Japanese once and repeating shampooing 360 times was used as the hair for evaluation in all cases.

The oily hair cosmetic composition of the respective Examples and Comparative Examples was applied to hair at the bath ratio shown in the tables, and then the hair was thoroughly dried without rinsing using a hair dryer. Those in which the hair was then washed once using the plain shampoo described below and dried was referred to as after shampooing once and those in which washing and drying was repeated 20 times were referred to as after shampooing 20 times.

In Comparative Example 5, the hair prepared by washing untreated damage hair once using the plain shampoo and then drying was used for evaluation (evaluation after shampooing 20 times was not performed).

| | |
|---|---|
| • Composition of plain shampoo (pH 6.9) | (% by mass) |
| Sodium polyoxyethylene lauryl ether sulfate (EMAL 170J manufactured by Kao Corporation, 70% by mass of active ingredient) | 13.0 |
| Coconut oil fatty acid monoethanolamide (AMISOL CME manufactured by Kawaken Fine Chemicals Co., Ltd.) | 0.6 |
| Coconut oil fatty acid amidopropylcarbobetaine (AMPHITOL 55AB manufactured by Kao Corporation, 30% by mass of active ingredient) | 1.41 |
| Citric acid | in amount to adjust pH |
| Sodium benzoate | 0.3 |
| Purified water | Balance |

### (Advancing contact angle of hair)

The advancing contact angle of hair with water was measured and determined as the value of contact angle. The advancing contact angle was measured using distilled water and a measurement apparatus, Processor Tensiometer K100 manufactured by KRUSS GmbH under conditions of a surface tension of water: 72.8 mN/m, a density of water: 0.998 g/cm³, a maximum immersion depth: 4 mm, a minimum immersion depth: 1 mm and a measurement speed: 2 mm/min. 5 hairs were collected from a hair tress which had been treated by the respective treatments, and the portion 5 cm from the root was used for the measurement. The average value of the five hairs was determined as the value of the contact angle. When the value is more than 90°, a higher value indicates that the hair is more hydrophobic. When the value is less than 90°, a lower value indicates that the hair is more hydrophilic. In short, a higher value of more than 90° indicates that the treatment successfully gives excellent hydrophobicity.

### (Combing force)

The combing force of hair after rinsing with warm water at 40°C for 15 seconds was measured by the dynamic combing force method (Suzuki, et. al., J. Soc. Cosmet. Chem. Japan. Vol. 27, No. 1, P11-13 1993). The average of 10 measured values was employed. A smaller value indicates that combability is better and the hair is smoother.

### (Sensory evaluation)

The items of "easy untangling of tress in drying step," "good finger combability during drying," "little stickiness of hair to fingers after drying," and "quick drying of hair" were evaluated by 7 expert panelists. The compositions were evaluated on a scale of 1 to 5 below using the total score.

"Easy untangling of tress in drying step"
- 1:: Untangling very bad
- 2:: Untangling bad
- 3:: Neither

- 4:: Untangling good
- 5:: Untangling very good

"Good finger combability during drying"
- 1:: Finger combability very bad
- 2:: Finger combability bad
- 3:: Neither
- 4:: Finger combability good
- 5:: Finger combability very good

"Little stickiness of hair to fingers after drying"
- 1:: Very sticky
- 2:: Sticky
- 3:: Neither
- 4:: Less sticky
- 5:: Little sticky

"Quick drying of hair"
- 1:: Very difficult to dry
- 2:: Difficult to dry
- 3:: Neither
- 4:: Dry quickly
- 5:: Dry very quickly

**[Table 1]**

| Content (% by mass; all are active ingredents) | | Example | | | | | | Comparative Example | | | | |
|---|---|---|---|---|---|---|---|---|---|---|---|---|
| | | 1 | 2 | 3 | 4 | 5 | 6 | 1 | 2 | 3 | 4 | 5(*2) |
| (A) | Epoxyaminosilane copolymer (*1) | 1 | 1 | 1 | 1 | - | 1 | 1 | 1 | 1 | 1 | - |
| | Aminoprapyltriethoxysilane | - | - | - | - | 1 | - | - | - | - | - | - |
| (B) | Isocbdecane (*3) | 93.3 | - | - | - | 99 | 20 | - | - | - | - | - |
| | Hydrogenated polyisobutene(*4) | - | 93.3 | - | - | - | - | - | - | - | - | - |
| | Dimethyl polysiloxane (*5) | - | - | 93.3 | - | - | - | - | - | - | - | - |
| | Methyltrimethicone (*6) | - | - | - | 93.3 | - | - | - | - | - | - | - |
| | Isopropyl palmitate (*7) | - | - | - | - | - | - | - | - | - | - | - |
| | Benzyl alcohol | - | - | - | - | - | - | - | - | - | - | - |
| (B') | Glycerol | - | - | - | - | - | - | 93.3 | - | - | - | - |
| | Ethylene glycol | - | - | - | - | - | - | - | 93.3 | - | - | - |
| | Triethylene glycol | - | - | - | - | - | - | - | - | 93.3 | - | - |
| (C) | Ethanol | - | - | - | - | - | - | - | - | - | - | - |
| Others | Water (*8) | 5.7 | 5.7 | 5.7 | 5.7 | - | 5.7 | 5.7 | 5.7 | 5.7 | 99 | - |
| | Dimethyl ether (propellant) | - | - | - | - | - | 73.3 | - | - | - | - | - |
| Total amount | | 100 | 100 | 100 | 100 | 100 | 100 | 100 | 100 | 100 | 100 | - |
| Treatment bath ratio (aqueous solution / hair) | | 0.51 | 0.5 | 0.5 | 0.5 | 0.5 | 0.5 | 0.5 | 0.51 | 0.5 | 0.5 | - |
| Advancing contact angle (°) | After shampooing once | 128 | 122 | 130 | 131 | 98 | 118 | 96 | 99 | 111 | 116 | 59 |
| | After shampooing 20 times | 106 | 94 | 111 | 109 | 91 | 99 | 61 | 62 | 77 | 79 | |
| Combing force | After shampooing once | 189 | 199 | 181 | 188 | 241 | 228 | 422 | 389 | 372 | 366 | 728 |
| | After shampooing 20 times | 222 | 231 | 199 | 201 | 282 | 243 | 699 | 671 | 666 | 626 | - |
| Sensory evaluation (After shampooing once) | Easy untangling of tress in drying step | 31 | 30 | 33 | 32 | 29 | 30 | 20 | 19 | 21 | 20 | 7 |
| | Good timer combability during drying | 31 | 30 | 33 | 32 | 27 | 28 | 18 | 19 | 20 | 20 | 7 |
| | Little stickiness of hair to fingers after drying | 30 | 29 | 32 | 33 | 28 | 28 | 14 | 14 | 16 | 16 | 22 |
| | Quick drying of hair | 31 | 33 | 33 | 33 | 29 | 29 | 13 | 14 | 15 | 15 | 7 |
| Sensory evaluation (After shampooing 20 times) | Easy untangling of tress in drying step | 30 | 30 | 32 | 31 | 27 | 29 | 13 | 14 | 16 | 15 | 7 |
| | Good finger combability during drying | 30 | 30 | 32 | 30 | 27 | 27 | 13 | 14 | 12 | 12 | 7 |
| | Little stickiness of hair to fingers after drying | 32 | 29 | 31 | 32 | 26 | 27 | 14 | 14 | 16 | 15 | 22 |
| | Quick drying of hair | 29 | 31 | 32 | 31 | 27 | 28 | 13 | 12 | 11 | 13 | 7 |

**[Table 2]**

| Content (% by mass; all are active ingredents) | | Example | | | | | | | | | | | | | | |
|---|---|---|---|---|---|---|---|---|---|---|---|---|---|---|---|---|
| | | 7 | 8 | 9 | 10 | 11 | 12 | 13 | 14 | 15 | 16 | 17 | 18 | 19 | 20 | 21 |
| (A) | Epoxyaminosilane copolymer(*1) | 1 | 1 | 1 | 1 | 1 | 1 | 1 | 1 | 1 | 0.1 | 0.25 | 0.5 | 2 | 2.28 | 5 |
| (B) | Isododecane(*3) | - | - | 50 | 30 | 10 | 5 | - | - | 50 | 50 | 50 | 50 | 50 | 50 | 50 |
| | Hydrogenated polyisobutene (*4) | - | - | - | - | - | - | - | - | 20 | - | - | - | - | - | - |
| | Dimethyl polysiloxane (*5) | - | - | - | - | - | - | - | - | - | - | - | - | - | - | - |
| | Methyltrimethicone(*6) | - | - | - | - | - | - | - | - | - | - | - | - | - | - | - |
| | Isopropyl palmitate (*7) | 83.3 | 73.3 | - | - | - | - | - | - | - | - | - | - | - | - | - |
| | Benzyl alcohol | - | - | - | - | - | - | 93.3 | 50 | - | - | - | - | - | - | - |
| (B') | Glycerol | - | - | - | - | - | - | - | - | - | - | - | - | - | - | - |
| | Ethylene glycol | - | - | - | - | - | - | - | - | - | - | - | - | - | - | - |
| | Triethylene glycol | - | - | - | - | - | - | - | - | - | - | - | - | - | - | - |
| (C) | Ethanol | 10 | 20 | 43.3 | 63.3 | 83.3 | 88.3 | - | 43.3 | 23.3 | 49.33 | 48.32 | 46.65 | 36.6 | 34.72 | 16.5 |
| Others | Water (*8) | 5.7 | 5.7 | 5.7 | 5.7 | 5.7 | 5.7 | 5.7 | 5.7 | 5.7 | 0.57 | 1.43 | 2.85 | 11.4 | 13 | 28.5 |
| Total amount | | 100 | 100 | 100 | 100 | 100 | 100 | 100 | 100 | 100 | 100 | 100 | 100 | 100 | 100 | 100 |
| Treatment bath ratio (Aqueous solution/hair) | | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 |
| Advancing contact angle (°) | After shampooing once | 122 | 125 | 129 | 132 | 133 | 130 | 128 | 130 | 122 | 118 | 118 | 121 | 129 | 129 | 132 |
| | After shampooing 20 times | 103 | 104 | 110 | 114 | 108 | 111 | 104 | 109 | 100 | 92 | 97 | 99 | 113 | 114 | 111 |
| Carting force | After shampooing once | 196 | 199 | 187 | 156 | 182 | 179 | 191 | 181 | 171 | 199 | 199 | 194 | 186 | 190 | 188 |
| | After shampooing 20 times | 241 | 234 | 203 | 180 | 210 | 209 | 209 | 210 | 208 | 239 | 230 | 219 | 200 | 197 | 211 |
| Sensory evaluation (After shampooing once) | Easy untangling of tress in drying step | 29 | 29 | 33 | 34 | 34 | 33 | 32 | 33 | 32 | 29 | 31 | 31 | 31 | 31 | 30 |
| | Good finger combability during drying | 30 | 31 | 35 | 35 | 34 | 33 | 32 | 31 | 31 | 31 | 33 | 33 | 34 | 34 | 33 |
| | Little stickiness of hair to fingers after drying | 28 | 29 | 31 | 33 | 33 | 32 | 31 | 30 | 30 | 30 | 30 | 31 | 31 | 31 | 30 |
| | Quick drying of hair | 29 | 29 | 34 | 33 | 33 | 33 | 32 | 32 | 30 | 30 | 30 | 31 | 32 | 32 | 34 |
| Sensory evaluation (After shampooing 20 times) | Easy untangling of tress in drying step | 28 | 29 | 31 | 33 | 33 | 33 | 30 | 31 | 30 | 28 | 30 | 31 | 31 | 32 | 30 |
| | Good finger combability during drying | 29 | 29 | 33 | 34 | 32 | 32 | 30 | 29 | 30 | 29 | 30 | 30 | 32 | 32 | 33 |
| | Little stickiness of hair to fingers after drying | 28 | 28 | 30 | 32 | 31 | 32 | 29 | 29 | 28 | 29 | 30 | 30 | 31 | 31 | 30 |
| | Quick drying of hair | 28 | 28 | 29 | 31 | 30 | 30 | 29 | 29 | 28 | 28 | 29 | 30 | 31 | 31 | 33 |

*1: Silsoft CLX-E (manufactured by Momentive Performance Materials, polysilicone-29, 15% by mass)
*2: Untreated damaged hair
*3: MARUKASOL R (manufactured by Maruzen Petrochemical Co., Ltd.)
*4: PARLEAM 3 (manufactured by NOF Corporation)
*5: KF-96L-0.65cs (manufactured by Shin-Etsu Silicone Co., Ltd.)
*6: TMF-1.5 (manufactured by Shin-Etsu Silicone Co., Ltd.)
*7: EXCEPARL IPP (manufactured by KAO CORPORATION)
*8: Including water derived from Silsoft CLX-E and dipropylene glycol

## Claims

1. A hair cosmetic composition comprising the following components (A) and (B), wherein the content of component (A) is 0.05% by mass or more and 10% by mass or less, and the content of component (B) is 5% by mass or more and 99.9% by mass or less:
(A) a self-crosslinking compound; and
(B) a compound having an IOB of 1.3 or less in the organic conceptual diagram and being liquid at 20°C.

2. The composition for a fiber treatment agent according to claim 1, wherein the component (A) is one or more selected from the group consisting of the following components (A1) and (A2):
(A1) an alkoxysilyl group-containing silicone; and
(A2) an alkoxysilyl group-containing alkylamine.

3. The hair cosmetic composition according to claim 2, wherein the component (A1) is an epoxyaminosilane copolymer, which is a reaction product of the following compounds (a) to (d):
(a) a polysiloxane having at least two oxiranyl groups or oxetanyl groups;
(b) a polyether having at least two oxiranyl groups or oxetanyl groups;
(c) aminopropyltrialkoxysilane; and
(d) a compound selected from the group consisting of the following primary and secondary amines:
• primary amines: methylamine, ethylamine, propyleneamine, ethanolamine, isopropylamine, butylamine, isobutylamine, hexylamine, dodecylamine, oleylamine, aniline, aminopropyltrimethylsilane, aminopropyltriethylsilane, aminomorpholine, aminopropyldiethylamine, benzylamine, naphthylamine, 3-amino-9-ethylcarbazole, 1-aminoheptafluorohexane, and 2,2,3,3,4,4,5,5,6,6,7,7,8,8,8-pentadecafluoro-1-octanamine; and
• secondary amines: methylethylamine, methyloctadecylamine, diethanolamine, dibenzylamine, dihexylamine, dicyclohexylamine, piperidine, pyrrolidinephthalimide, and a polymer amine.

4. The hair cosmetic composition according to any one of claims 1 to 3, further comprising the following component (C):
(C) an aliphatic alcohol having 1 to 4 carbon atoms.

5. The hair cosmetic composition according to claim 3 or 4, wherein the compound (a) is a compound of the following formula (1): 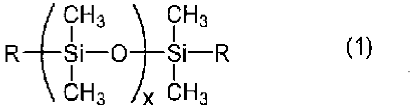 wherein R represents a hydrocarbon group having 1 to 6 carbon atoms and having an oxiranyl group or an oxetanyl group at the terminal thereof and optionally having an oxygen heteroatom, and x represents a number of from 1 to 1,000.

6. The hair cosmetic composition according to any one of claims 3 to 5, wherein the compound (b) is a compound of the following formula (2): 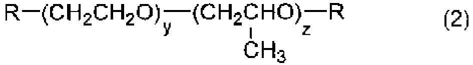 wherein R is as defined above, y is a number of from 1 to 100, z is a number of from 0 to 100, and y + z is a number of from 1 to 200.

7. The hair cosmetic composition according to any one of claims 3 to 6, wherein the alkoxy group in the compound (c) has 1 to 6 carbon atoms.

8. The hair cosmetic composition according to any one of claims 3 to 7, wherein the compound (c) is at least one selected from the group consisting of aminopropyltrimethoxysilane, aminopropyltriethoxysilane, aminopropyltripropoxysilane, aminopropyltriisopropoxysilane, aminopropyltributoxysilane and aminopropyl-tri-tert-butoxysilane.

9. The hair cosmetic composition according to any one of claims 3 to 8, wherein the compound (d) is a primary amine.

10. The hair cosmetic composition according to any one of claims 1 to 9, wherein the component (A) is Polysilicone-29.

11. The hair cosmetic composition according to any one of claims 1 to 10, wherein the component (B) is at least one selected from the group consisting of isododecane, hydrogenated polyisobutene, mineral oil, jojoba oil, octyldodecyl isostearate, octyldodecyl neopentanoate, toriisostearin, isodecyl neopentanoate, dimethylpolysiloxane, phenyltrimethicone, methyltrimethicone, isopropyl palmitate, hydrogenated castor oil, dimethyl ether, glyceryl isostearate, benzyl alcohol, phenoxyethanol, PEG 20 hydrogenated castor oil, PEG 25 hydrogenated castor oil, PEG 30 hydrogenated castor oil and PEG 45 hydrogenated castor oil.

12. The hair cosmetic composition according to any one of claims 1 to 11, wherein the content of water is 13% by mass or less.

13. A method of using the hair cosmetic composition according to any one of claims 1 to 12, comprising applying the hair cosmetic composition to hair and then drying the hair without rinsing off.

14. The method of using the hair cosmetic composition according to claim 13, wherein the amount of the hair cosmetic composition to be applied to hair is determined relative to the mass of the hair so that the bath ratio is 0.001 or more and 100 or less.
